# EUROPEAN PATENT SPECIFICATION

(11) **EP 2 702 967 B1**
(45) Date of publication and mention of the grant of the patent: **22.03.2017**
(21) Application number: 12776696.2
(22) Date of filing: 25.04.2012
(51) Int. Cl.: A61F 9/007, A61F 2/16

(54) **INTRAOCULAR LENS IMPLANTATION TOOL**
WERKZEUG ZUM EINSETZEN EINER KONTAKTLINSE
OUTIL D'IMPLANTATION DE LENTILLE INTRAOCULAIRE

(30) Priority: 28.04.2011 JP 2011102482; 28.04.2011 JP 2011102483
(43) Date of publication of application: 05.03.2014
(73) Proprietor: Nidek Co., Ltd., Gamagori-shi, Aichi 443-0038 (JP)
(72) Inventor: NAGASAKA Shinji, Gamagori-shi Aichi 443-0038 (JP)
(74) Representative: Prüfer & Partner mbB Patentanwälte · Rechtsanwälte
(86) International application number: PCT/JP2012/061121
(87) International publication number: WO 2012/147810

(56) References cited:
- EP-A2- 2 574 308
- EP-A2- 2 599 460
- WO-A1-2006/070561
- WO-A1-2008/029498
- JP-A- 2002 514 466
- JP-A- 2003 325 570
- JP-A- 2005 131 147
- JP-A- 2005 169 107
- JP-A- 2007 503 872
- JP-A- 2010 017 459
- US-A1- 2010 130 985

## Description

### TECHNICAL FIELD

The present invention relates to an intraocular lens (IOL) injection instrument for injecting an IOL into an eye.

### BACKGROUND ART

As an injector to be used for injection of an IOL, there is known a preset type injector that is stored and delivered, in which an IOL has been previously set in the injector. In this type of injector, a holding member (a holding means) to restrain movement of an IOL during storage is installed as a separate component and will be detached in use to enable use of the IOL (see Patent Document 1).

### RELATED ART DOCUMENTS

### PATENT DOCUMENTS

Patent Document 1: JP 2008-61677A

### SUMMARY OF INVENTION

### PROBLEMS TO BE SOLVED BY THE INVENTION

However, when the holding member (the holding means) for holding the IOL is configured as a separate component from an injector body as disclosed in Patent Document 1, the injector body has a complicated internal configuration. Furthermore, a work to detach the holding member has to be performed first when the IOL is to be used. This leads to a problem with a troublesome work.

The present invention has been made to solve the above problems and has a purpose to provide an IOL injection instrument capable of holding an IOL with a simple configuration and of appropriately releasing the IOL from a held state in use.

### MEANS OF SOLVING THE PROBLEMS

The above-mentioned object is achieved by an IOL injection instrument comprising the features of claim 1. Some advantageous configurations of the invention are disclosed in the dependent claims.

### EFFECTS OF THE INVENTION

An injector capable of holding and releasing an IOL can be provided with a simple configuration.

### BRIEF DESCRIPTION OF THE DRAWINGS

FIG. 1 is a perspective external view of an IOL injection instrument;
FIG. 2 is an explanatory view showing a configuration of a push-out member;
FIG. 3A is a side view of the IOL injection instrument;
FIG. 3B is another side view of the IOL injection instrument;
FIG. 4A is an explanatory view showing a configuration of an IOL holder;
FIG. 4B is another explanatory view showing the configuration of the IOL holder;
FIG. 4C is another explanatory view showing the configuration of the IOL holder;
FIG. 5A is an explanatory view showing a releasing operation of the IOL from a held state;
FIG. 5B is another explanatory view showing the releasing operation of the IOL from the held state; and
FIG. 5C is another explanatory view showing the releasing operation of the IOL from the held state.

### MODE FOR CARRYING OUT THE INVENTION

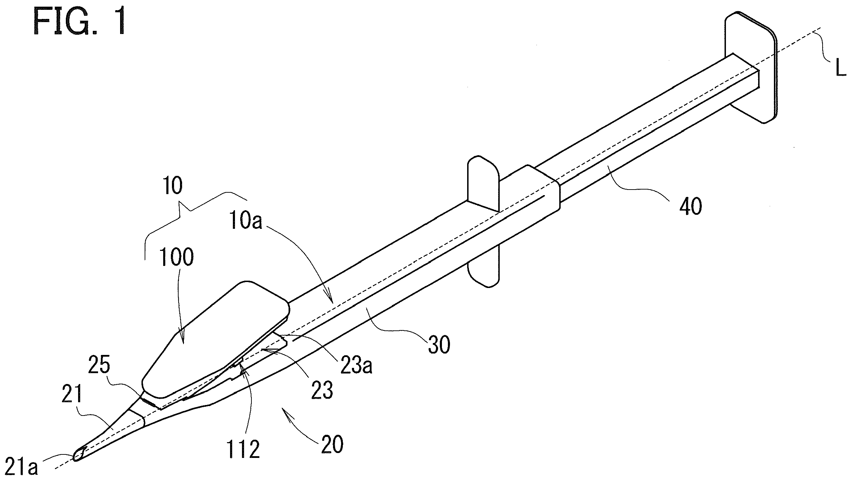

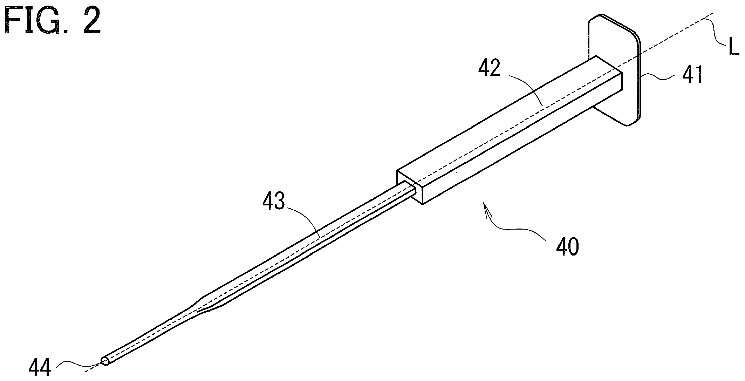

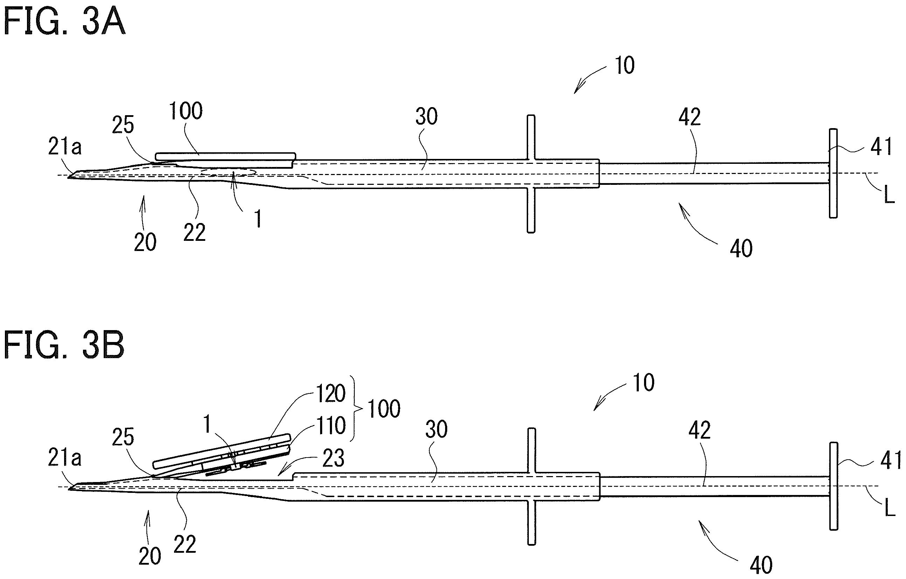

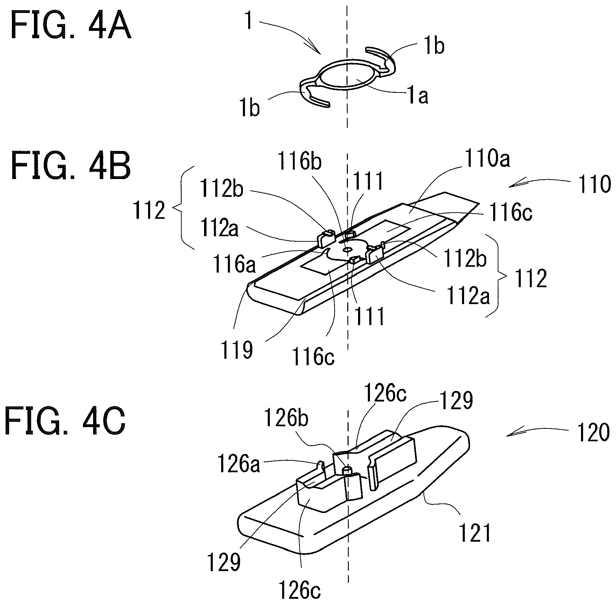

A detailed description of an embodiment of the present invention will now be given referring to the accompanying drawings. FIG. 1 is a perspective external view of an IOL injection instrument (hereinafter, referred to as an injector) 10. FIG. 2 is an explanatory view showing a configuration of a push-out member (hereinafter, referred to as a plunger) 40. FIGs. 3A and 3B are side views of the injector 10. FIGs. 4A to 4C are explanatory views showing a configuration of an IOL holder, illustrating perspective views of an intraocular lens (IOL) 1 and each constituent part.

The injector 10 consists of an injector body 10a for injection of the IOL 1 into an eye and an IOL holder 100 to hold (store) the unused IOL 1. The injector body 10a includes a hollow tubular injection part 20 to fold the IOL 1 into a smaller shape, a hollow cylinder body 30 provided with the injection part 20 at a front end, and a plunger 40 attached to be movably in a front-back direction with respect to the cylinder body 30 to push out the IOL 1 into an eye.

The injection part 20 includes an injection tube 21 having a nearly cylindrical shape formed with a front end 21 a, a setting part 22 on which the IOL 1 will be placed in use, and an opening 23 through which the IOL 1 held in the IOL holder 100 will pass toward the setting part 22. The setting part 22 is provided on a basal end side of the injection part 20 and the opening 23 is provided near the setting part 22. In the present embodiment, the opening 23 is assumed to be located above the setting part 22. The injection tube 21 is formed in a taper shape having an internal diameter gradually decreasing (becoming narrower) toward the front end 21a. The opening 23 is designed in such a size (shape) that causes no defect in the IOL 1 such as breakage during movement of the IOL 1 held in the IOL holder 100 into the injector body 10a. For instance, an edge 23a of the opening 23 in this embodiment is provided in a position beyond the range enabling the IOL 1 pushed out from the IOL holder 100 to pass in an open state (an unstressed state). The IOL put on the setting part 22 through the opening 23 is pushed by the plunger 40 to pass through the inside of the injection tube 21 and is folded into a smaller shape along the shape of an inner wall of the injection tube 21, and then injected out through the front end 21 a.

The IOL holder 100 is attached rotatably to the injector body 10a through a joint 25 provided in the injection tube 21 at a position close to the front end 21a. On a side of the IOL holder 100 which will be brought in contact (be connected) with the opening 23, a cover 110 is prepared to serve as holding means formed in such a shape that the cover 110 and the opening 23 close each other (see FIG. 3B). Accordingly, when the IOL holder 100 is turned or rotated in a direction to come close to the injector body 10a to close the opening 23, the inside of the injector body part 10a is sealed. Even though not illustrated, the cover 110 and the opening 23 are closed to each other through an engagement portion.

On the other hand, when the IOL holder 100 is rotated in a direction to come away from the injector body 10a, the opening 23 is opened, making the setting part 22 (or the inner wall of the injection tube 21 which will contact with the IOL 1) appear to enable application of a viscoelastic material to the setting part 22 through the opening 23.

Further, the cover 110 (the side to be closed to the opening 23) of the IOL holder 100 includes holding part 111 and 112 serving as restriction means (see FIG. 4B), whereby the IOL 1 is held outside a push-out axis L of the plunger 40. The push-out axis L of the plunger 40 is an axis along which the plunger 40 is moved forward and backward and forth in a front-back direction in pushing the IOL 1 into an eye. Accordingly, when the cover 110 and the opening 23 are closed to each other, the inside of the injector body 10a is sealed and also the IOL 1 is placed on the setting part 22 outside the push-out axis L. The details of the configuration of the IOL holder 100 will be explained later.

The joint 25 used herein is a strong component enough to enable rotation of the IOL holder 100 to open and close with respect to the injector body 10a. For instance, the joint 25 may be a well-known hinge or the like. Another alternative is to connect the injector body 10a and the IOL holder 100 with a thin bent part made of the same material as them.

The plunger 40 includes a press part 41 to be pressed by an operator, an axial base part 42 connected to the press part 41, a push rod 43 connected to the axial base part 42, and a tip 44 continuous to the push rod 43 and to be brought into contact with an optic part of the IOL 1 in pushing out the IOL 1. The thus configured plunger 40 is inserted to move forward and backward in a path formed throughout the cylinder body 30 up to the front end of the injection part 20 along the push-out axis L.

The configuration of the IOL holder 100 will be explained below. In FIGs. 4A to 4C, the IOL holder 100 is formed of a combination of the 110 for holding the IOL 1 and a push means 120 including a plurality of push-out parts to release holding of the IOL 1 held in the cover 110 and push the IOL 1 onto the setting part 22. It is to be noted that FIGs. 4A to 4C illustrate that the IOL 1 held in the cover 110 is placed with the back side of a lens face facing upward (an inverted position from that shown in FIG. 1),

In FIG. 4A, the IOL 1 is illustrated as a one-piece type IOL having an optic part 1a and a pair of support parts 1b, which are integrally made of soft material. Alternatively, the IOL 1 may also be selected from well-known IOLs foldable by the injector 10. For instance, the IOL may be a three-piece type IOL formed of an optic part and a pair of support parts separately produced and then assembled into one.

A mounting surface 110a of the cover 100 on the side facing to the injector body 10a is provided with holding parts 111 and 112 each extending in a columnar shape in a vertical direction with respect to the mounting surface 110a to hold the IOL 1. The holding parts 111 are formed in positions along (in contact with) the outer peripheral shape of the IOL 1 in order to restrain horizontal movement of the IOL 1 with respect to the mounting surface 110a. Herein, the holding parts 111 are formed at two places corresponding to joining positions of the optic part 1a and the support parts 1b.

The holding parts 112 are provided in right and left positions (in a perpendicular direction to the push-out direction) with respect to the optic part 1a so as to touch the outer edge of the optic part 1a (or slightly outside the optic part 1a) placed on the mounting surface 110a, Each of the holding parts 112 consists of a columnar support part 112a extending in the vertical direction with respect to the mounting surface 110a and a restriction member 112b attached to extend inward at an approximately right angle from a leading end of the support part 112a. Each restriction member 112b serves to restrict movement of the IOL 1 (the optic part 1 a) placed on the mounting surface 110a in the pushing direction defined by push parts mentioned later to hold the IOL 1 (the optic part 1a) outside the push-out axis L. Each restriction member 112b is formed in a protruding shape extending from a surrounding area of the optic part 1a toward the inside (herein, the center of the optic part) by a predetermined length in a horizontal direction.

The above configured cover 110 is made of resin. Each restriction member 112b is formed with such a thickness as to provide flexibility to allow deformation in the moving direction of the IOL 1 by pressing force applied by the push means 120 mentioned later. It is to be noted that the restriction members 112b are formed with a thickness (a wall thickness) providing flexibility and strength so that each restriction member 112b is not deformed by the weight of the IOL 1 or the force generated by the movement (slight movement) of the IOL 1 on the mounting surface 110a, while each restriction member 112b is easily deformed (bent about a connected position with each support part 112a) by pushing force applied from the push means 120. Further, the length of each restriction member 112b is determined such that a distal end of each restriction member 112b deformed when the IOL 1 is released from holding by the holding parts 112 does not reach the push-out axis L of the plunger 40.

With the above configured holding parts 112, the restriction members 112b restrain the up and down movement of the IOL 1 with respect to the mounting surface 110a during storage of the IOL 1 and while the push operation of the push means 120 mentioned later is not yet performed. While the IOL holder 100 and the opening 23 are closed to each other, the IOL 1 may be held by the restriction members 112b in a position slightly apart from the mounting surface 110a.

On the other hand, when the restriction members 112b are applied by pressing force through the IOL 1 by the press-in operation of the push member 120 in use of the IOL 1, the restriction members 112b are turned (deformed) outward (along the moving direction of the IOL). Accordingly, the IOL 1 is allowed to pass through the previous positions of the restriction members 112b before deformation and thus the IOL 1 is moved and released from holding by the holding parts 112.

Since the restriction members 112b are flexible to be deformed, even if the peripheral edge of the optic part 1a touches the restriction members 112b when the IOL 1 is moved toward the injector body 10a, the peripheral edge of the optic part 1a is less likely to be damaged, e.g., scratched, by stress applied by the touch.

Specifically, the restriction members 112b for holding the IOL 1 are formed to be deformable, so that the optic part is less likely to be subjected to unnecessary stress and the IOL 1 is appropriately released from the holding. The restriction members 112b have only to be configured so that at least a part thereof which will contact with the optic part 1a is deformable. For instance, the restriction members 112b are formed with such a thickness a shape as to allow deformation from the connected positions with the support parts 112a.

In the present embodiment as explained above, the restriction members 112b provided in the IOL holder 100 restrain the movement of the optic part 1a in the right-left direction and the movement of the same in an up-down direction to come close to or apart from the mounting surface 110a. This prevents the IOL 1 from moving within the IOL holder 100 during storage, thereby avoiding erroneous push-out toward the injector body 10a.

The height of the holding parts 111 from the mounting surface 110a are determined so that the holding parts 111 come into contact with the outer periphery of the stored IOL 1. In the holding parts 112 (the support portions 112a), the restriction members 112b are provided in positions higher than the height (thickness) of a lens face (herein, a back surface) of the IOL 1 placed on the mounting surface 110a. Accordingly, the IOL 1 is stably held in an unstressed state outside the push-out axis L. On the other hand, as the length of each holding part 111 and 112 is shorter, a push amount of the push means 120 is shorter in releasing the IOL 1 from holding.

Furthermore, the height (length) of each holding part 111 and 112 is determined to be high (long) not to extend to the push-out axis L (injection axis) of the plunger 40 when the opening 23 is closed by the cover 110. While the cover 110 and the injector body 10a remain closed to each other, accordingly, the IOL 1 is appropriately folded by the plunger 40.

In a case where the length of each restriction member 112b is sufficiently short as not to reach the push-out axis L, after the restriction members 112b are deformed outward once by pressing force of the push member 120, the restriction members 112b do not need to return to original states. Of course, the restriction members 112b may be returned to the original states by their own restoring force after they are deformed.

In the cover 110, at a position corresponding to the edge 23a of the opening 23, a stepped groove 119 is formed to fit with the edge 23a when the opening 23 is closed by the cover 110, thereby sealing the inside of the injector body 10a.

The cover 110 is further formed with a plurality of through holes 116 for allowing a plurality of push-out parts of the push means 120. In the present embodiment, there are provided a through hole 116a formed in a nearly circular arc shape corresponding to the position of the peripheral edge of the optic part 1a placed in the cover 110, a through hole 116b formed in a nearly circular shape corresponding to the center position of the optic part 1a, and through holes 116c formed in rectangular shape corresponding to each position of the support parts 1b. Since the through holes 116a-c are formed to correspond to each part of the IOL 1 and to allow the push-out parts to pass through, thereby pushing the whole IOL 1 in a balanced manner (uniformly). The through holes 116a-c are required only to be formed in at least one place allowing the push-out parts to push out the IOL 1 and thus formed in a position corresponding to at least one of the optic part and the support parts.

The push means 120 is a member for pushing the IOL 1 from the IOL holder 100 toward the injector body 10a and includes a plate 121 by which the operator can apply pushing force and push-out parts 126a-c provided in correspondence to the positions of the aforementioned through holes 116a-c. The push means 120 is made of resin as with the cover 110.

The push-out parts 126a-c are designed such that their leading ends are formed in shapes nearly equal to (slightly smaller than) the shapes of the through holes 116a-c to pass through the through holes 116a-c. Accordingly, when the pushing force is applied through the plate 121, the push-out parts 126a-c are caused to pass through the through holes 116a-c into contact with the IOL 1. To hold a positional relationship between the cover 110 and the push means 120 during storage of the IOL 1, the push-out parts 126a-c in the present embodiment are formed with width (diameter) gradually increasing from the leading ends to base ends (the plate 121). While the pushing force is not applied to the plate 121, therefore, the predetermined positional relationship between the push means 120 and the cover 110 is maintained. As an alternative, a fitting part such as protrusions and recesses may be provided in the cover 110 and the push means 120 to hold the positional relationship between the cover 110 and the push means 120 during storage of the IOL 1.

The push-out parts 126a-c each have a length enough to deform the restriction members 112b through the optic part 1a but not enough to reach the push-out axis L of the plunger 40 when the push-out parts 126a-c are pushed into contact with the cover 110 from the state where the cover 110 and the opening 23 are closed to each other to fully push the IOL 1 into the injector body 10a. Thus, after pushing of the IOL 1, it is possible to push out the IOL 1 into an eye by a single operation without needing removal of the push means 120.

In the leading ends (contact surfaces) of the push-out parts 126c which will contact with the support parts 1b, there are formed grooves 129 each extending in a front-back direction (the push-out axis L of the plunger 40) of the IOL 1 placed in the cover 110. Each groove 129 is formed in a shape nearly coincident with the outer shape (diameter) of the push rod 43 of the plunger 40, and used as a centering mechanism of the plunger in use of the IOL 1. The leading end (contact surface) of each push-out part 126a which will contact with the optic part 1a (a front surface side) is preferably formed with a curved surface (a round shape) having an area as large as possible and conforming to the shape of the optic part 1a. Thus, the load exerted on the optic part 1a can be appropriately removed when the IOL 1 is folded by the plunger 40.

With the above configuration, during storage, the IOL 1 is held in an unstressed state by the plurality of holding parts of the IOL holder 100. In use, the IOL can be moved toward the injector body 10a (onto the setting part 22) by a simple operation that pushes the push means 120 from a state where the opening 23 is closed by the cover 110. Furthermore, the rotation operation to rotate the IOL holder 100 so that the cover 110 closes the opening 23 and the push operation to move the IOL 1 from the IOL holder 100 to the setting part 22 are performed in a series of motions (a single operation).

Meanwhile, to reduce frictional resistance generated between the IOL and the inner wall of the injector when the IOL is to be pushed out, it is assumed preferable to apply a viscoelastic material to the inner wall surface (a bottom surface and others) of the injector which will contact with the IOL. However, in JP 2003-325570A, an IOL is sealed in an injector and thus the viscoelastic material could not be applied to an inner wall surface of the injector. In JP 2008-61677A, a cover is used to open the inside of an injector; however, an IOL is placed in advance on a bottom surface and thus the viscoelastic material could not be applied. In JP 2007-503872A, an IOL is held by a separate component from an injector body. This needs, in addition to the above operation to apply the viscoelastic material, works to attach the IOL to the injector body and place the IOL into a push-out enabled state in the injector body. This configuration is thus troublesome.

In the present embodiment, on the other hand, the viscoelastic material can be easily applied to the setting part 22 through the opening 23 remaining opened after rotation of the IOL holder 100. Furthermore, the viscoelastic material is spread uniformly over in the injection tube 21 in association with an operation that closes the IOL holder 100 and the opening 23 to each other and push the IOL 1 toward the injector body 10a (in association with the movement of the IOL 1 to the placement part 22). Therefore, applying of the viscoelastic material and setting of the IOL can be more efficiently performed than in a conventional art.

The injector 10 configured as above is made of resin by molding. For instance, the injector body 10a and the IOL holder 100 are separately made by molding and then connected to each other through the joint 25. In the IOL holder 100, the cover 110 and the push means 120 are individually made by molding. The restriction members 112b of the holding parts 112 are made integral with the cover 110 (the support parts 112a) by molding or may be bonded to the cover 110 (the support parts 112a) previously produced. As still another alternative, the restriction members 112b may be produced by cutting work, including machining the cover 110.

The push-out operation of the IOL 1 using the injector 10 configured as above will be explained below. Herein, the rotating operation of the IOL holder 100 is explained referring to the side views of the injector 10 in FIGs. 3A and 3B. In a state where the IOL 1 is held, in FIG. 3A, the opening 23 is closed by the cover 110 to seal the inside of the injection tube 21. At that time, the IOL 1 is held outside the push-out axis L by the holding parts 111 and 112 (the restriction members 112b). Herein, the IOL 1 is placed facing downward (the back surface faces downward) in the injector body 10a. However, the holding parts 111 and 112 are not deformed by the weight and the vibration of the IOL 1, so that the IOL 1 is stably held in the injector body 10a by the holding parts 111 and 112.

Subsequently, the operator releases the IOL 1 from holding outside the push-out axis L. The operator first rotates or turns the IOL holder 100 to come away from the injector body 10a as shown in FIG. 3B in order to apply the viscoelastic material to the setting part 22 (the inner wall of the injection tube 21). The opening 23 is thus opened, making the setting part 22 (the inner wall of the side with which the IOL will contact) appears. In this state, the operator applies the viscoelastic material to the setting part 22 by use of a known syringe or the like. In the present embodiment, since the opening 23 is being widely open for application of the viscoelastic material, the operator is able to easily (uniformly) apply the viscoelastic material to the setting part 22.

After completion of application of the viscoelastic material, the operator pushes the plate 121 of the push means 120 to rotate the IOL holder 100 back toward the injector body 10a to seal the inside of the injector body 10a again. Accordingly, the groove 119 is fitted with the edge 23a of the opening 23 and thus the cover 110 closes the opening 23 (see FIG. 3A). Further, when the plate 121 of the push means 120 is continuously pressed while the opening 23 is closed, the push-out parts 126a-c of the push means 120 gradually pass through the Through holes 116a-c of the cover 110. When the leading ends of the push-out parts 126a-c contact with the IOL 1, the IOL 1 is pushed by pushing force applied from the push means 120. At that time, the push-out parts 126a-c are brought into uniform contact with the whole IOL 1. The IOL 1 is thus pushed stably toward the injector body 10a.

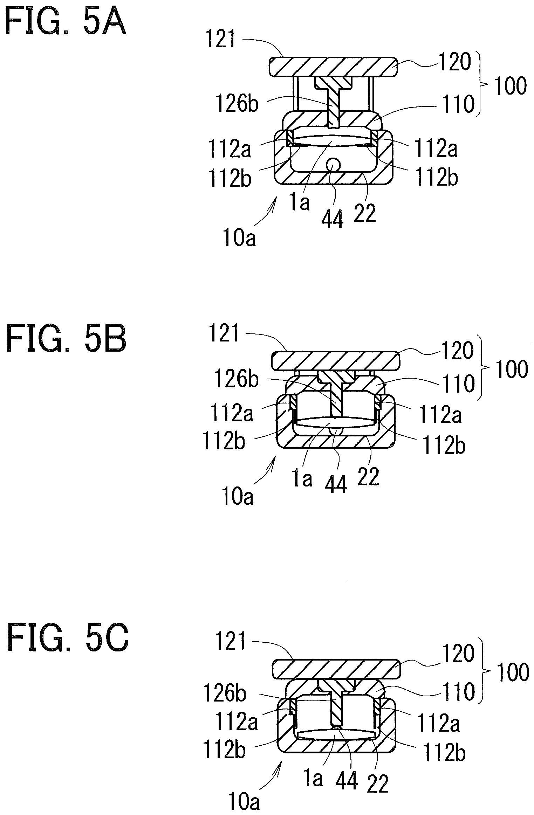

As the IOL 1 is pushed toward the injector body 10a, the IOL 1 is gradually released from the holding by the holding parts 111 and 112. Herein, FIGs. 5A to 5C are explanatory views showing an operation of releasing the IOL 1 from the holding. It is to be noted that FIGs. 5A to 5C are cross sectional views of the IOL holder 100 taken in the position of the holding part 112. For convenience of explanation, the push-out parts 126a, 126c, and others are not illustrated.

While the IOL 1 is held in the holding parts 112 and 111 as shown in FIG. 5A, when the IOL 1 is pushed through the push-out parts 126a-c (only the push-out part 126b is illustrated in FIGs. 5A to 5C) by pushing force applied to the plate 121, the push-out parts 126a-c come into contact with the IOL 1 (the front surface side of the optic part 1a). When the plate 121 is further pushed in, as shown in FIG. 5B, the restriction members 112b are gradually deformed outward by the pushing force through the IOL 1 (the optic part 1a). When the IOL 1 is further pushed in, the restriction members 112b are further deformed outward along the moving direction of the IOL 1. As the IOL 1 is moved, passing the previous positions of the restriction members 112b before deformation, toward the injector body 10a (onto the push-out axis L), the IOL 1 is released from the holding. In this embodiment, it is assumed that the length of each restriction member 112b is set sufficiently short and the restriction members 112b are held as deformed after passage of the IOL 1. When the push means 120 is further pushed in, as shown in FIG. 5C, the IOL 1 is placed on the setting part 22 of the injector body 10a (on the push-out axis L).

As above, the lens surface of the IOL 1 is held in a direction that the IOL 1 is pushed and moved, and the holding parts to be deformed by pressing force of the push-out parts 126a-c are provided, so that holding of the IOL 1 is easily released by a push operation. Since the holding parts 111 and 112 are located in positions not reaching the push-out axis L of the plunger 40 when the IOL 1 is placed on the setting part 22, the operator can continue to make a folding operation of the IOL without removing the IOL holder 100.

On the other hand, when the push means 120 is completely pushed in the cover 110 and the IOL 1 is placed on the setting part 22, the grooves 129 formed in the contact surfaces of the push-out parts 126c are located on the push-out axis L of the plunger 40 (the push rod 43) and used as a centering mechanism. Specifically, the folding operation of the IOL 1 can be appropriately performed without removing the push means 120 pushed in the cover 110. As explained above, moreover, the viscoelastic material is appropriately spread over in the injection tube 21 as the IOL 1 is placed on the setting part 22 applied in advance with the viscoelastic material, thereby reducing frictional resistance between the IOL 1 and the inner wall in pushing out the IOL 1. The IOL 1 is easily placed on the setting part 22 in association with rotation of the push member 120 as explained above.

The operator successively performs an operation to inject the IOL 1 into an eye of a patient. When the press part 41 is pushed by the operator, the tip 44 of the plunger 40 is brought in contact with the peripheral edge of the optic part 1a. As the press part 41 is further pushed, the optic part 1a is gradually folded (rounded) along the inner wall of the injection tube 21. At that time, the movement of the plunger 40 in the front-back direction is made stable by the centering by the groove 129, so that the optic part 1a is stably folded. When the press part 41 is still further pushed, the IOL 1 is moved ahead, as being folded, toward the front end 21a.

When the optic part 1a is fed out from the front end 21a as above, the optic part 1a is gradually opened in a capsule. When the tip 44 of the plunger 40 is moved up to the front end 21 a of the injection tube 21, the rear-side support part 1b is also moved into the eye. The pair of support parts 1b are then placed along the capsule, so that the optic part 1a is held by the stress applied from the inside of the eye.

With the above configuration in the present embodiment, the IOL injection instrument of a type holding an IOL in advance enables easy application of a viscoelastic material. Further, from the state where the viscoelastic material is applied in advance, the operation to seal the inside of the injector body 10a and the operation to move the IOL 1 onto the setting part can be performed by a single operation. It is further possible to hold the IOL by use of simply configured holding parts during storage of the IOL and easily release the IOL from the holding in sync with the push-out operation.

The above explanation exemplifies the injector configured such that the injector body and the IOL holder are integrally formed (a preset type or preload type injector), but the invention is not limited thereto. For instance, when the above injector configuration is applied to a case where an injector body and an IOL holder are separately provided, a viscoelastic material can be easily applied to a required portion. Further, holding and using of an IOL can be easily changed over. For instance, the configuration of the present invention is applicable to an injector arranged such that an IOL holder constitutes a part of an injector body (the IOL holder forms a part of an inner wall of an injection part of the injector body), that is, a semi-preset type or semi-preload type injector.

In the aforementioned configuration, the IOL holder connected to the injector body is rotatable; however, the present invention is not limited thereto. For instance, a slide part may be provided to support an IOL holder slidably in an up-down direction with respect to an opening of an injector body. This configuration enables easy application of a viscoelastic material from the position of the opening while an IOL is held in the IOL holder. In this case, when the IOL holder is pushed in toward the injector body, coming close to the injector body through the slide part, the opening may be closed by the IOL holder (a cover).

Although the aforementioned configuration exemplifies that the opening is formed above the setting part, the opening may be provided in a side (in a lateral direction) with respect to the setting part. Besides, the opening has only to be provided in any position (near the setting part) if only applying a viscoelastic material to the setting part is enabled while the IOL is held in the IOL holder. In the above explanation, the whole injector 10 is sealed when the opening 23 is closed by the IOL holder 100 (the cover 110). As an alternative, the IOL holder 100 and the injector body 10a may be separately sealed.

The above configuration shows an example that the IOL holder 100 prepared as a separate component from the injector body 10a holds the IOL 1 outside the push-out axis L of the plunger 40. Besides, even in a case where the IOL 1 is to be held on the push-out axis L of the plunger 40, a restriction member may also be formed to restrain movement of the IOL in a front-back direction on the push-out axis L. In this case, a restriction member is provided in a position which will be placed in contact with a part (edge) of the optic part in association with the push-out operation of the plunger 40 and which is off the push-out axis L of the plunger 40, the restriction member being to be deformed by a force applied by slight movement such as vibration of the IOL 1 but being not to be deformed by pressing force applied from the plunger 40 (it is noted that the configuration of the restriction member is identical to the above and is not illustrated herein). Thus, during storage, the restriction member can restrain movement of the IOL 1 in the direction of the push-out axis L (the front-back direction). During use, on the other hand, the restriction member is deformed by the pressing force applied by the push-out operation of the plunger 40 through the IOL 1. Accordingly, the IOL 1 can be easily released from the held state without being under load. Since the restriction member is provided in a position off the push-out axis L, releasing of the IOL from the held state and pushing-out of the IOL into an eye can be continuously performed by only the push-out operation of the IOL without removing the holding part.

In a case where the IOL 1 held in the IOL holder 100 is to be pushed in the injector body 10a in the lateral direction (from side), a restriction member to be deformed by pressing force applied by the push-out member has only to be provided in the moving direction of the IOL 1. Specifically, in the moving direction to push the IOL 1 from the held state to a set state (a usable state), a restriction member configured as with the above one may be formed.

The configuration that the IOL holder is detachable from the injector body as in the above embodiment may also be used as an injector of a type in which an IOL is loaded later by an operator as well as the above preset type injector. This configuration enables one type of injector to be used for different purposes.

### REFERENCE SIGNS LIST

- 1: Intraocular lens (IOL)
- 1a: Optic part
- 1b: Support part
- 10: IOL injection instrument, esp. injector
- 10a: Injector body
- 20: Injection part
- 21: Injection tube
- 21a: Front end
- 22: Setting part
- 23: Opening
- 23a: Edge
- 25: Joint
- 30: Cylinder body
- 40: Push-out member, esp. plunger
- 41: Press part
- 42: Axial base
- 43: Push rod
- 44: Tip
- 100: IOL holder
- 110: Holding means, esp. cover
- 110a: Mounting surface
- 111: Holding part
- 112: Holding part
- 112a: Support part
- 112b: Restriction member
- 116a-c: Through hole
- 119: Groove
- 120: Push means
- 121: Plate
- 126a-c: Push-out part
- 129: Groove
- L: Push-out axis

## Claims

1. An intraocular lens (IOL) injection instrument (10) including:
an injector body (10a) which includes:
an injection part (20) having an inner wall to fold an IOL (1) into a smaller shape; and
a cylinder body (30) including a push-out member (40) attached to be movable in a front-back direction with respect to the cylinder body (30) along a push-out axis (L) to push out the IOL (1) from a front end (21 a) of the injection part (20) along the push-out axis (L),
holding means (110) movably attached to the injector body (10a) to hold the IOL (1) without applying stress thereto; and
push means (120) in combination with the holding means (110) for pushing the IOL (1) by applying pressing force to the IOL to release holding of the IOL held by the holding means (110) and to pass the IOL (1) onto a setting part (22) of the injection part (20) so that the IOL (1) put on the setting part (22) can be pushed by the push-out member (40) along the push-out axis (L),
**characterized in that**
the holding means (110) includes a restriction member (112b) configured to restrict movement of the IOL (1) and to hold the IOL (1) in a position outside the push-out axis (L) while no pressing force is applied by the push means (120) and configured to be deformed in a moving direction of the IOL perpendicular to the push-out axis (L) when the pressing force is applied by the push means (120) to release restriction of the movement of the IOL.

2. The IOL injection instrument according to claim 1, wherein
the restriction member (112b) comprises a protruding member made of resin, and the protruding member extends in a plane parallel to the push-out axis (L) of the push-out member (40) to support the IOL (1) and has a thickness allowing deformation in the moving direction of the IOL (1) perpendicular to the push-out axis (L) by the pressing force by the push means (120).

3. The IOL injection instrument according to claim 2, wherein the protruding member is dimensioned such that it is placed outside the push-out axis (L) of the push-out member (40) when the IOL (1) is released from holding by the holding means (110).

4. The IOL injection instrument according to any one of preceding claims, wherein
the injection part (20) further includes an opening (23) provided near the setting part (22) to allow the IOL (1) to be put on the setting part (22) through the opening (23), and the holding means (110) is movable with respect to the injection part (20) to place the IOL (1) on the setting part (22) and close the opening (23).

5. The IOL injection instrument according to claim 4, wherein the holding means (110) is a cover being provided above the setting part (22) and being rotatable to close the opening (23).

6. The IOL injection instrument according to claim 5, wherein the push means (120) is held on the holding means (110) to perform both a rotation operation of the holding means (110) to close the opening (23) and a push operation of the IOL (1) to the setting part (22) after the opening (23) is closed by the holding means (110).

7. The IOL injection instrument according to any one of preceding claims, wherein
the push means (120) includes a push-out part (126a-c) to push the IOL (1) held by the holding means (110) onto the setting part (22),
the holding means (110) has a through hole (116a-c) to allow the push-out part (126a-c) to pass through and contact with the IOL (1), and
the push-out part (126a-c) passes through the through hole (116a-c) to push the IOL (1) when the push means (20) is pushed with respect to the holding means (110), so that the IOL (1) is released from restriction by the restriction member (112b).

8. The IOL injection instrument according to claim 7, wherein the push-out part (126a-c) of the push means (20) and the through hole (116a-c) of the holding means (110) are formed in positions corresponding to the IOL (1) held by the restriction member (112b) of the holding means (110).

9. The IOL injection instrument according to claim 8, wherein the push-out part (126a-126c) comprises a leading end coming into contact with the IOL (1), wherein the leading end is formed with a groove (129) extending in a front-back direction of the instrument (10) to make centering of the push-out member (40).

10. The IOL injection instrument according to any one of claims 4 to 9, wherein the opening (23) of the injection part (20) is formed to be larger than an outer shape of the IOL (1) in an unstressed state.

## Patentansprüche

1. Intraolcularlinsen (IOL) - Injektionsinstrument (10), mit einem Injektorkörper (10a), der enthält:
ein Injektionsteil (20) mit einer Innenwand, um eine IOL (1) in eine kleinere Form zusammenzulegen; und
einen Zylinderkörper (30) mit einem Ausschubelement (40), das in einer Vorne-Hinten-Richtung bezüglich des Zylinderkörpers (30) entlang einer Ausschubachse (L) bewegbar angebracht ist, um die IOL (1) aus einem vorderen Ende (21a) des Injektionsteils (20) entlang der Ausschubachse (L) herauszuschieben;
einer Halteeinrichtung (110), die bewegbar an dem Injektorkörper (10a) angebracht ist, um die IOL (1) zu halten ohne sie zu belasten; und
einer Druckeinrichtung (120) in Kombination mit der Halteeinrichtung (110) zum Drücken der IOL (1) durch Ausüben einer Druckkraft auf die IOL, um das Halten der durch die Halteeinrichtung (110) gehaltenen IOL zu lösen und die IOL (1) auf ein Einführteil (22) des Injektionsteils (20) zu überführen, sodass die auf das Einführteil (22) gesetzte IOL (1) durch das Ausschubelement (40) entlang der Ausschubachse (L) geschoben werden kann,
**dadurch gekennzeichnet, dass**
die Halteeinrichtung (110) ein Beschränkungselement (112b) enthält, das ausgestaltet ist, um eine Bewegung der IOL (1) zu beschränken und die IOL (1) in einer Position außerhalb der Ausschubachse (L) zu halten, wenn keine Druckkraft auf die Druckeinrichtung (120) ausgeübt wird, und ausgestaltet ist, um in einer Bewegungsrichtung der IOL senkrecht zur Ausschubachse (L) verformt zu werden, wenn die Druckkraft durch die Druckeinrichtung (120) ausgeübt wird, um die Beschränkung der Bewegung der IOL freizugeben.

2. IOL-Injektionsinstrument nach Anspruch 1, bei welchem
das Beschränkungselement (112b) ein vorstehendes Element aus Kunstharz aufweist, und
sich das vorstehende Element in einer Ebene parallel zur Ausschubachse (L) des Ausschubelements (40) erstreckt, um die IOL (1) zu halten, und eine Dicke hat, die eine Verformung in der Bewegungsrichtung der IOL (1) senkrecht zur Ausschubachse (L) durch die Druckkraft durch die Druckeinrichtung (120) erlaubt.

3. IOL-Injektionsinstrument nach Anspruch 2, bei welchem
das vorstehende Element so dimensioniert ist, dass es außerhalb der Ausschubachse (L) des Ausschubelements (40) platziert ist, wenn die IOL (1) vom Halten durch die Halteeinrichtung (110) gelöst ist.

4. IOL-Injektionsinstrument nach einem der vorhergehenden Ansprüche, bei welchem
das Injektionsteil (20) ferner eine Öffnung (23) enthält, die nahe dem Einführteil (22) vorgesehen ist, um die IOL (1) durch die Öffnung (23) auf das Einführteil (22) setzen zu können, und
die Halteeinrichtung (110) bezüglich des Injektionsteils (20) bewegbar ist, um die IOL (1) auf dem Einführteil (22) zu platzieren und die Öffnung (23) zu schließen.

5. IOL-Injektionsinstrument nach Anspruch 4, bei welchem
die Halteeinrichtung (110) ein Deckel ist, der über dem Einführteil (22) vorgesehen ist und drehbar ist, um die Öffnung (23) zu schließen.

6. IOL-Injektionsinstrument nach Anspruch 5, bei welchem
die Druckeinrichtung (120) an der Halteeinrichtung (110) gehalten ist, um sowohl ein Drehbetätigung der Halteeinrichtung (110) zum Schließen der Öffnung (23) als auch eine Druckbetätigung der IOL (1) zum Einführteil (22) nach dem Schließen der Öffnung (23) durch die Halteeinrichtung (110) durchzuführen.

7. IOL-Injektionsinstrument nach einem der vorhergehenden Ansprüche, bei welchem
die Druckeinrichtung (120) ein Ausrückteil (126a-c) enthält, um die durch die Halteeinrichtung (110) auf dem Einführteil (22) gehaltene IOL (1) auszurücken,
die Halteeinrichtung (110) ein Durchgangsloch (116a-c) hat, um das Ausrückteil (126a-c) hindurch passieren und mit der IOL (1) in Kontakt kommen zu lassen, und
das Ausrückteil (126a-c) durch das Durchgangsloch (116a-c) hindurch passiert, um die IOL (1) auszurücken, wenn die Druckeinrichtung (20) bezüglich der Halteeinrichtung (110) gedrückt wird, sodass die IOL (1) von der Beschränkung durch das Beschränkungselement (112) gelöst wird.

8. IOL-Injektionsinstrument nach Anspruch 7, bei welchem
das Ausrückteil (126a-c) der Druckeinrichtung (20) und das Durchgangsloch (116a-c) der Halteeinrichtung (110) an Positionen entsprechend der durch das Beschränkungselement (112b) der Halteeinrichtung (110) gehaltenen IOL (1) ausgebildet sind.

9. IOL-Injektionsinstrument nach Anspruch 8, bei welchem
das Ausrückteil (126a-c) ein vorderes Ende aufweist, das mit der IOL (1) in Kontakt kommt, wobei das vordere Ende mit einer Nut (129) ausgebildet ist, die in einer Vorne-Hinten-Richtung des Instruments (10) verläuft, um das Ausschubelement (40) zu zentrieren.

10. IOL-Injektionsinstrument nach einem der Ansprüche 4 bis 9, bei welchem
die Öffnung (23) des Injektionsteils (20) größer als eine Außenform der IOL (1) im unbelasteten Zustand ausgebildet ist.

## Revendications

1. Outil d'implantation de lentille intraoculaire (LIO) (10) comportant :
un corps de dispositif d'implantation (10a) qui comporte :
une partie d'implantation (20) présentant une paroi interne pour plier une LIO (1) sous une forme plus petite ; et
un corps cylindrique (30) comportant un élément de poussée (40) fixé pour être mobile dans une direction avant/arrière par rapport au corps cylindrique (30) le long d'un axe de poussée (L) pour pousser la LIO (1) à partir d'une extrémité avant (21a) de la partie d'implantation (20) le long de l'axe de poussée (L),
un moyen de maintien (110) fixé de manière mobile au corps de dispositif d'implantation (10a) pour maintenir la LIO (1) sans appliquer de contrainte sur celle-ci ; et
un moyen de poussée (120) en combinaison avec le moyen de maintien (110) pour pousser la LIO (1) en appliquant une force de pression sur la LIO pour libérer le maintien de la LIO maintenue par le moyen de maintien (110) et pour faire passer la LIO (1) sur une partie de réglage (22) de la partie d'implantation (20) de manière à ce que la LIO (1) installée sur la partie de réglage (22) puisse être poussée par l'élément de poussée (40) le long de l'axe de poussée (L),
**caractérisé en ce que**
le moyen de maintien (110) comporte un élément de restriction (112b) configuré pour restreindre le mouvement de la LIO (1) et pour maintenir la LIO (1) dans une position à l'extérieur de l'axe de poussée (L) sans aucune application de force de pression par le moyen de poussée (120) et configuré pour être déformé dans une direction de déplacement de la LIO perpendiculaire à l'axe de poussée (L) lorsque la force de pression est appliquée par le moyen de poussée (120) pour libérer la restriction du mouvement de la LIO.

2. Outil d'implantation de LIO selon la revendication 1, dans lequel
l'élément de restriction (112b) comprend un élément en saillie composé de résine, et
l'élément en saillie s'étend dans un plan parallèle à l'axe de poussée (L) de l'élément de poussée (40) pour supporter la LIO (1) et présente une épaisseur permettant une déformation dans la direction de déplacement de la LIO (1) perpendiculaire à l'axe de poussée (L) par la force de pression par le moyen de poussée (120).

3. Outil d'implantation de LIO selon la revendication 2, dans lequel l'élément en saillie est dimensionné de telle sorte qu'il est placé à l'extérieur de l'axe de poussée (L) de l'élément de poussée (40) lorsque la LIO (1) est libérée du maintien par le moyen de maintien (110).

4. Outil d'implantation de LIO selon l'une quelconque des revendications précédentes, dans lequel la partie d'implantation (20) comporte en outre une ouverture (23) disposée près de la partie de réglage (22) pour permettre à la LIO (1) d'être installée sur la partie de réglage (22) à travers l'ouverture (23), et le moyen de maintien (110) est mobile par rapport à la partie d'implantation (20) pour placer la LIO (1) sur la partie de réglage (22) et fermer l'ouverture (23).

5. Outil d'implantation de LIO selon la revendication 4, dans lequel le moyen de maintien (110) est un couvercle disposé au-dessus de la partie de réglage (22) et étant rotatif pour fermer l'ouverture (23).

6. Outil d'implantation de LIO selon la revendication 5, dans lequel le moyen de poussée (120) est maintenu sur le moyen de maintien (110) pour réaliser à la fois une opération de rotation du moyen de maintien (110) pour fermer l'ouverture (23) et une opération de poussée de la LIO (1) sur la partie de réglage (22) après la fermeture de l'ouverture (23) par le moyen de maintien (110).

7. Outil d'implantation de LIO selon l'une quelconque des revendications précédentes, dans lequel
le moyen de poussée (120) comporte une partie de poussée (126a-c) pour pousser la LIO (1) maintenue par le moyen de maintien (110) sur la partie de réglage (22),
le moyen de maintien (110) présente un trou traversant (116a-c) pour permettre à la partie de poussée (126a-c) de traverser la LIO (1) et d'entrer en contact avec celle-ci, et
la partie de poussée (126a-c) traverse le trou traversant (116a-c) pour pousser la LIO (1) lorsque le moyen de poussée (20) est poussé par rapport au moyen de maintien (110), afin que la LIO (1) soit libérée de la restriction par l'élément de restriction (112b).

8. Outil d'implantation de LIO selon la revendication 7, dans lequel la partie de poussée (126a-c) du moyen de poussée (20) et le trou traversant (116a-c) du moyen de maintien (110) sont formés dans des positions correspondant à la LIO (1) maintenue par l'élément de restriction (112b) du moyen de maintien (110).

9. Outil d'implantation de LIO selon la revendication 8, dans lequel la partie de poussée (126a-126c) comprend une extrémité avant entrant en contact avec la LIO (1), dans lequel l'extrémité avant est formée avec une rainure (129) s'étendant dans une direction avant/arrière de l'outil (10) pour effectuer le centrage de l'élément de poussée (40).

10. Outil d'implantation de LIO selon l'une quelconque des revendications 4 à 9, dans lequel l'ouverture (23) de la partie d'implantation (20) est formée pour être plus large qu'une forme externe de la LIO (1) dans un état non contraint.
